# EUROPÄISCHE PATENTSCHRIFT

(11) **EP 1 118 072 B1**
(45) Veröffentlichungstag und Bekanntmachung des Hinweises auf die Patenterteilung: **30.10.2002**
(21) Anmeldenummer: 99955801.8
(22) Anmeldetag: 24.09.1999
(51) Int. Cl.: G09F 9/33, H05B 33/00

(54) **VERFAHREN ZUM HERSTELLEN EINES BAUELEMENTES UND BAUELEMENT**
METHOD FOR PRODUCING A COMPONENT AND CORRESPONDING COMPONENT
PROCEDE POUR PRODUIRE UN ELEMENT ET ELEMENT AINSI OBTENU

(30) Priorität: 30.09.1998 DE 19845075
(43) Veröffentlichungstag der Anmeldung: 25.07.2001
(73) Patentinhaber: Osram Opto Semiconductors GmbH & Co. OHG, 93049 Regensburg (DE)
(72) Erfinder: HEIMGÄRTNER, Rudolf, D-93047 Regensburg (DE)
(74) Vertreter: Epping Hermann & Fischer
(86) Internationale Anmeldenummer: DE9903083
(87) Internationale Veröffentlichungsnummer: WO00019397

(56) Entgegenhaltungen:
- US-A- 4 486 499
- US-A- 4 849 674
- US-A- 5 804 917

## Beschreibung

Die Erfindung betrifft ein Verfahren zum Herstellen eines Bauelementes aus wenigstens einem organischen, elektrolumineszierenden Funktionselement und einer Glasscheibe, wobei das Funktionselement im Bereich einer Innenfläche der Glasscheibe angeordnet wird.

Die Erfindung betrifft ferner ein Bauelement aus wenigstens einem organischen, elektrolumineszierenden Funktionselement und einer Glasscheibe, wobei das organische, elektrolumineszierende Funktionselement im Bereich einer Hauptfläche der Glasscheibe angeordnet ist.

Bei einem bekannten gattungsgemäßen Verfahren wird das Funktionselement zwischen zwei parallel ausgerichteten Glasscheiben angeordnet. Anschließend werden die Glasscheiben miteinander verklebt. Hierbei tritt jedoch das Problem auf, daß Feuchtigkeit über die Klebeschicht in den Hohlraum diffundiert.

Weiterhin ist aus US 5,804,917 bekannt, auf einem Glassubstrat eine organische, elektrolumineszierende Schichtstruktur aufzubringen und diese mit einem Decksubstrat abzudecken, wobei das Decksubstrat und das Glassubstrat mittels eines Versiegelungsmittels verbunden sind, das die organische Schichtstruktur rahmenartig umgibt.

Der Erfindung liegt die Aufgabe zugrunde, die Nachteile des Standes der Technik zu überwinden. Insbesondere soll ein mit möglichst geringem Aufwand durchführbares Verfahren geschaffen werden, mit dem ein gattungsgemäßes Bauelement so hergestellt werden kann, daß das Funktionselement vor äußeren Einflüssen möglichst wirksam geschützt ist.

Erfindungsgemäß wird diese Aufgabe dadurch gelöst, daß die im fertigen Bauelement eine Innenfläche bildende Hauptfläche der Glasscheibe mittels eines Glaslots so mit einem Rahmen verbunden wird, daß der Rahmen im fertigen Zustand des Bauelementes Randflächen des Funktionselementes umgibt.

Die Erfindung sieht vor, auf der Glasscheibe einen Rahmen aufzubringen, der mit der Glasscheibe durch Verlöten mit einem Glaslot verbunden wird..

Es ist besonders vorteilhaft, das erfindungsgemäße Verfahren so durchzuführen, daß zuerst die Glasscheibe mit dem Rahmen verbunden wird und daß dann das Funktionselement im Bereich der Hauptfläche der Glasscheibe angeordnet wird.

Besonders vorteilhaft ist es, daß die Leiterstruktur auf der Hauptfläche aufgebracht wird, bevor die Hauptfläche mit dem Rahmen verbunden wird.

Die erfindungsgemäße Technologie ist besonders da geeignet, da das Funktionselement wenigstens eine organische lichtemittierende Diode (OLED) enthält.

Das Material der Glasscheibe und des Rahmens werden zweckmäßigerweise so gewählt, daß ihre thermischen Ausdehnungskoeffizienten weitgehend aneinander angepaßt sind. Eine solche Anpassung erfolgt beispielsweise durch eine Änderung der chemischen Zusammensetzung des Glases oder durch eine geeignete Wahl des Rahmenmaterials.

Wenn jedoch aus prozeßtechnischen Gründen ein Material für den Rahmen gewählt wird, das einen anderen Ausdehnungskoeffizienten aufweist als die Glasscheibe, was beispielsweise bei einem Metallrahmen der Fall ist, dann muß die Verbindung zwischen der Glasscheibe und dem Rahmen so beschaffen sein, daß sie unterschiedliche thermische Ausdehnungen ausgleicht.

Als Glaslote werden solche Gläser bezeichnet, die infolge besonders niedriger Schmelztemperatur eine Glaslotverbindung zwischen der Glasscheibe und dem mit ihr zu verbindenden Körper, das heißt hier dem Rahmen, ermöglichen. Das Glaslot wird so ausgewählt, daß ein hinreichendes Fließen und Benetzen des Glaslotes bei einer Temperatur stattfindet, bei der die Glasscheibe noch keine störenden Deformationen zeigt. Die Temperatur für den Lötvorgang entspricht im wesentlichen der Transformationstemperatur des Glaslotes.

Eine Anpassung des Glaslotes an die thermische Ausdehnung der Verbindungspartner ist wichtig, weil das Lot der mechanisch schwächere Verbindungspartner ist.

Eine zweckmäßige Durchführungsform dieses Verfahrens zeichnet sich dadurch aus, daß wenigstens ein Teil des Glaslotes auf die Glasscheibe aufgetragen wird und daß anschließend der Rahmen gegenüber der Glasscheibe positioniert wird.

Das Aufbringen des Glaslotes auf die Glasscheibe erfolgt durch Siebdrucken oder Dispensieren eines Stranges.

Eine weitere zweckmäßige Ausführungsform des Verfahrens zeichnet sich dadurch aus, daß wenigstens ein Teil des Glaslotes auf den Rahmen aufgebracht wird und daß anschließend der Rahmen gegenüber der Glasscheibe positioniert wird.

Um den Schutz des Funktionselementes vor äußeren Einflüssen zu verbessern, ist es zweckmäßig, daß der Rahmen mit einem Deckel verbunden wird.

Dies geschieht zweckmäßigerweise durch Verschweißen oder Verlöten.

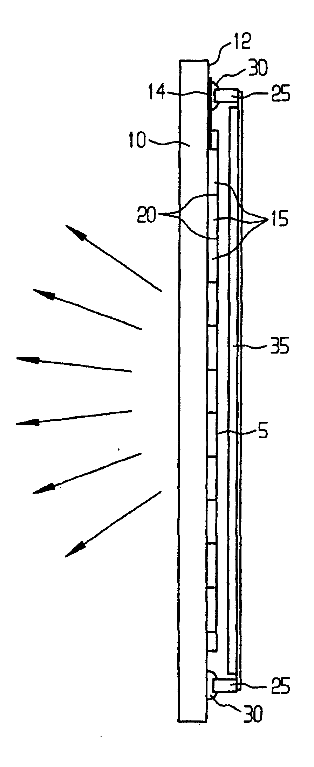

Weitere Vorteile, Besonderheiten und Weiterbildungen der Erfindung ergeben sich aus den Unteransprüchen sowie der nachfolgenden Darstellung eines bevorzugten Ausführungsbeispiels anhand der Zeichnung.

Die Zeichnung zeigt einen Querschnitt durch ein erfindungsgemäßes Bauelement aus einem organischen, elektrolumineszierenden Funktionselement 5 und einer Glasscheibe 10. Das Funktionselement 5 befindet sich auf einer Hauptfläche 12 der Glasscheibe 10.

Bei dem Funktionselement 5 handelt es sich um eine Anzeigeeinheit (Display), die aus einer Vielzahl von in einem zweidimensionalen Feld angeordneten, Licht emittierenden organischen Dioden (OLED) besteht.

Die einzelnen LEDs 15 weisen mehrere Funktionsschichten auf, wie sie beispielsweise in dem Artikel von M. Schwoerer und H. v. Seggern: Electroluminescent Chromophores and Polymeres for Organic LEDs and Displays, Siemens-Review Special; R & D, Herbst 1996, S. 20 ff. dargestellt ist.

Die einzelnen LEDs 15 bilden zusammen ein Display, wobei die einzelnen LEDs jeweils einem Pixel (= Picture Element = Bildelement) entsprechen. Ein Pixel ist ein zweidimensionales Bildelement, beispielsweise ein Quadrat.

Die LEDs 15 bestehen aus mehreren Funktionsschichten und befinden sich im Bereich einer ihrer Hauptflächen in einem unmittelbaren Kontakt mit der Glasscheibe 10.
Zwischen den LEDs 15 befinden sich Stege 20.

Auf der Hauptfläche 12 der Glasscheibe 10 ist ein das Funktionselement 5 auf allen Seitenflächen umgebender Rahmen 25 angeordnet. Der Rahmen 25 besteht beispielsweise aus einer geeigneten Metallegierung oder aus einem keramischen Material.

Zwischen dem Rahmen 25 und der Hauptfläche 12 der Glasscheibe 10 befindet sich ein Glaslot 30.

Auf dem Rahmen 25 befindet sich ein Deckel 35. Der Deckel 35 besteht beispielsweise aus einer geeigneten Metallegierung.

Das dargestellte Bauelement wird vorzugsweise wie folgt hergestellt:

Auf der Hauptfläche 12 der Glasscheibe 10 werden Leiterstrukturen 14, beispielsweise in Form von Leiterbahnen, durch den Auftrag geeigneter Materialien gebildet. Hierzu-ist es besonders zweckmäßig, ein Kathodenzerstäubungsverfahren (Sputterverfahren) einzusetzen.

Anschließend wird auf die Hauptfläche 12 der Glasscheibe 10 ein Glaslot, beispielsweise das im Handel unter der Bezeichnung DM 2700 P erhältliche Glaslot, aufgetragen.

Alternativ ist es möglich, das Glaslot auf dem Rahmen 25 in Bereichen, die zur Verbindung mit der Hauptfläche 12 der Glasscheibe 10 bestimmt sind, aufzutragen. Ein Aufbringen des Glaslotes 30 auf der Hauptfläche 12 der Glasscheibe 10 hat den Vorteil, daß es in einem einzigen Arbeitsgang, beispielsweise durch Siebdrucken oder Dispensieren des Glaslotes in Form eines Stranges erfolgen kann.

Zunächst werden der Rahmen 25 und die Glasscheibe 10 so zueinander positioniert, daß sich der Rahmen genau über Flächenbereichen befindet, auf denen zuvor das Glaslot aufgetragen wurde. Nachdem der Rahmen 25 oberhalb der durch das Glaslot 30 gebildeten Flächenbereiche positioniert wurde, wird der Rahmen 25 auf diese Bereiche abgesenkt.

Anschließend wird durch eine Erwärmung des Glaslotes auf seine Transformationstemperatur der Lötvorgang durchgeführt. Das hier eingesetzte Glaslot weist den besonderen Vorteil auf, daß es bereits bei einer für Lötvorgänge besonders niedrigen Temperatur von 350 Grad Celsius fließt und die zu verbindenden Flächen vollständig benetzt. Um sicher zu stellen, daß der Lötvorgang vollständig durchgeführt wurde, ist es vorteilhaft, daß der eigentliche Lötvorgang so erfolgt, daß der Lötbereich etwa 10 Minuten auf der Temperatur von 350 Grad Celsius gehalten wird.

Bei dem Lötvorgang benetzt das Glaslot 30 nicht nur die zu der Hauptfläche 12 zugewandten Flächen des Rahmens 25, sondern auch untere Bereiche von Seitenflächen des Rahmens 25. Das Auftreten dieses Benetzungsvorganges wird bei einer optischen Kontrolle erfaßt und ermöglicht es so, den vollständigen Abschluß des Lötvorganges nachzuweisen.

Anschließend wird das Funktionselement 5 innerhalb des Rahmens 25 auf die Hauptfläche 12 der Glasscheibe 10 aufgebracht.

Zum Erzielen eines wirksamen Abschlusses des Funktionselementes 5 vor äußeren Einflüssen wird der Rahmen 25 anschließend fest mit einem Deckel 35 verbunden. Diese Verbindung kann beispielsweise durch Verlöten oder Verschweißen erfolgen. Wenn sowohl der Rahmen 25 als auch der Deckel 30 aus einem Metall bestehen, ist ein Verschweißen besonders zweckmäßig.

Nachdem der Deckel 35 mit dem Rahmen 25 verbunden wurde, erfolgt eine Endkontrolle sowie eine Montage von elektrischen Anschlüssen.

### Bezugszeichenliste

- 5: optoelektronisches Funktionselement
- 10: Glasscheibe
- 12: Hauptfläche
- 14: Leiterstruktur
- 15: LED
- 20: Stege
- 25: Rahmen
- 30: Glaslot
- 35: Deckel

## Patentansprüche

1. Verfahren zur Herstellung eines Bauelementes aus wenigstens einem organischen, elektrolumineszierenden Funktionselement (5) und einer Glasscheibe (10), wobei das Funktionselement (5) im Bereich einer Hauptfläche (12) der Glasscheibe (10) angeordnet und die Hauptfläche (12) so mit einem Rahmen (25) verbunden wird, daß der Rahmen (25) im fertigen Zustand des Bauelementes die Randflächen des Funktionselementes (5) umgibt,
**dadurch gekennzeichnet,**
**daß** die Glasscheibe (10) mit dem Rahmen (25) durch Verlöten mit einem Glaslot (30) verbunden wird.

2. Verfahren nach Anspruch 1, **dadurch gekennzeichnet, daß** zuerst die Glasscheibe (10) mit dem Rahmen (25) verbunden wird und daß dann das Funktionselement (5) im Bereich der Hauptfläche (12) der Glasscheibe angeordnet wird.

3. Verfahren nach einem der Ansprüche 1 oder 2,
**dadurch gekennzeichnet,**
**daß** auf die Hauptfläche (12) eine Leiterstruktur (14) aufgebracht wird, bevor das Funktionselement (5) angeordnet wird.

4. Verfahren nach Anspruch 3, **dadurch gekennzeichnet, daß** die Leiterstruktur (14) auf der Hauptfläche (12) aufgebracht wird, bevor die Hauptfläche mit dem Rahmen (25) verbunden wird.

5. Verfahren nach einem der Ansprüche 1 bis 4,
**dadurch gekennzeichnet,**
**daß** das Funktionselement (5) auf der Hauptfläche (12) der Glasscheibe aufgebracht wird.

6. Verfahren nach einem der Ansprüche 1 bis 5,
**dadurch gekennzeichnet,**
**daß** wenigstens ein Teil des Glaslotes (30) auf die Glasscheibe (10) aufgetragen wird und daß anschließend der Rahmen (25) gegenüber der Glasscheibe positioniert wird.

7. Verfahren nach einem der Ansprüche 1 bis 6,
**dadurch gekennzeichnet,**
**daß** wenigstens ein Teil des Glaslotes (30) auf den Rahmen (25) aufgebracht wird und daß anschließend der Rahmen gegenüber der Glasscheibe (10) positioniert wird.

8. Verfahren nach einem der Ansprüche 1 bis 7,
**dadurch gekennzeichnet,**
**daß** der Rahmen (25) mit einem Deckel (35) verbunden wird.

9. Verfahren nach Anspruch 8,
**dadurch gekennzeichnet,**
**daß** der Deckel (35) mit dem Rahmen (25) durch Verschweißen verbunden wird.

10. Verfahren nach Anspruch 8,
**dadurch gekennzeichnet,**
**daß** der Deckel (35) mit dem Rahmen (25) durch Verlöten verbunden wird.

11. Bauelement aus wenigstens einem organischen, elektrolumineszierenden Funktionselement (5) und einer Glasscheibe (10), wobei das Funktionselement im Bereich einer Hauptfläche (12) der Glasscheibe (10) angeordnet und im Bereich der Seitenflächen von einem Rahmen (25) umgeben ist,
**dadurch gekennzeichnet,**
**daß** die Glasscheibe (10) und der Rahmen (25) mittels eines Glaslots (30) verbunden sind.

12. Bauelement nach Anspruch 11,
**dadurch gekennzeichnet,**
**daß** das Funktionselement wenigstens eine LED (15) enthält.

## Claims

1. Process for producing a component from at least one organic, electroluminescent functional element (5) and a glass pane (10), the functional element (5) being arranged in the region of a main surface (12) of the glass pane (10), and the main surface (12) being joined to a frame (25) in such a way that the frame (25), in the finished state of the component, surrounds the edge faces of the functional element (5), **characterized in that** the glass pane (10) is joined to the frame (25) by soldering using a soldering glass (30).

2. Process according to Claim 1, **characterized in that** first of all the glass pane (10) is joined to the frame (25), and **in that** then the functional element (5) is arranged in the region of the main surface (12) of the glass pane.

3. Process according to one of Claims 1 or 2, **characterized in that** a conductor structure (14) is applied to the main surface (12) before the functional element (5) is put in place.

4. Process according to Claim 3, **characterized in that** the conductor structure (14) is applied to the main surface (12) before the main surface is joined to the frame (25).

5. Process according to one of Claims 1 to 4, **characterized in that** the functional element (5) is applied to the main surface (12) of the glass pane.

6. Process according to one of Claims 1 to 5, **characterized in that** at least some of the soldering glass (30) is applied to the glass pane (10), and **in that** then the frame (25) is positioned with respect to the glass pane.

7. Process according to one of Claims 1 to 6, **characterized in that** at least some of the soldering glass (30) is applied to the frame (25), and **in that** then the frame is positioned with respect to the glass pane (10).

8. Process according to one of Claims 1 to 7, **characterized in that** the frame (25) is joined to a cover (35).

9. Process according to Claim 8, **characterized in that** the cover (35) is joined to the frame (25) by welding.

10. Process according to Claim 8, **characterized in that** the cover (35) is joined to the frame (25) by soldering.

11. Component comprising at least one organic, electroluminescent functional element (5) and a glass pane (10), the functional element being arranged in the region of a main surface (12) of the glass pane (10) and being surrounded by a frame (25) in the region of the side faces, **characterized in that** the glass pane (10) and the frame (25) are joined by means of a soldering glass (30).

12. Component according to Claim 11, **characterized in that** the functional element includes at least one LED (15).

## Revendications

1. Procédé de fabrication d'un élément constitué d'au moins un élément (5) fonctionnel organique électroluminescent et d'une vitre (10), l'élément (5) fonctionnel étant disposé dans la zone d'une surface (12) principale de la vitre (10) et la surface (12) principale étant reliée à un cadre (25) de façon à ce que la cadre (25) entoure, lorsque l'élément est à l'état fini, les surfaces de bord de l'élément (5) fonctionnel,
**caractérisé en ce que** la vitre (10) est reliée au cadre (25) par brasagee à l'aide d'une brasure (30) pour du verre.

2. Procédé suivant la revendication 1,
**caractérisé en ce que** l'on relie d'abord la vitre (10) au cadre (25) et **en ce que** l'on met ensuite l'élément (5) fonctionnel dans la zone de la surface (12) principale de la vitre.

3. Procédé suivant l'une des revendications 1 ou 2,
**caractérisé en ce que** l'on dépose sur la surface (12) principale une structure (14) conductrice avant de mettre l'élément (5) fonctionnel.

4. Procédé suivant la revendication 3,
**caractérisé en ce que** l'on dépose la structure (14) conductrice sur la surface (12) principale avant de relier la surface principale au cadre (25).

5. Procédé suivant l'une des revendications 1 à 4,
**caractérisé en ce que** l'on dépose l'élément (5) fonctionnel sur la surface (12) principale de la vitre.

6. Procédé suivant l'une des revendications 1 à 5,
**caractérisé en ce que** l'on dépose au moins une partie de la brasure (30) pour du verre sur la vitre (10) et **en ce que** l'on met ensuite le cadre (25) en position par rapport à la vitre.

7. Procédé suivant l'une des revendications 1 à 6,
**caractérisé en ce que** l'on dépose au moins une partie de la brasure (30) pour du verre sur le cadre (25) et ce que l'on met ensuite le cadre en position par rapport à la vitre (10).

8. Procédé suivant l'une des revendications 1 à 7,
**caractérisé en ce que** l'on relie le cadre (25) à un couvercle (35).

9. Procédé suivant la revendication 8,
**caractérisé en ce que** l'on relie le couvercle (35) au cadre (25) par soudage.

10. Procédé suivant la revendication 8,
**caractérisé en ce que** l'on relie le couvercle (35) au cadre (25) par brasage.

11. Elément composé au moins d'un élément (5) fonctionnel organique électroluminescent et d'une vitre (10), l'élément fonctionnel étant disposé dans la zone d'une surface (12) principale de la vitre (10) et étant entouré d'un cadre (25) dans la région des surfaces latérales,
**caractérisé en ce que** la vitre (10) et le cadre (25) sont reliés au moyen d'une brasure (30) pour du verre.

12. Elément suivant la revendication 11,
**caractérisé en ce que** l'élément fonctionnel comporte au moins une diode (15) électroluminescente.
